# EUROPÄISCHE PATENTSCHRIFT

(11) **EP 0 289 930 B1**
(45) Veröffentlichungstag und Bekanntmachung des Hinweises auf die Patenterteilung: **26.05.1993**
(21) Anmeldenummer: 88106748.2
(22) Anmeldetag: 27.04.1988
(51) Int. Cl.: C12P 21/08, C07K 15/00, C12N 5/12, G01N 33/577, G01N 33/68

(54) **Monoklonaler Antikörper zur selektiven immunologischen Bestimmung von intaktem Prokollagen Peptid (Typ III) und Prokollagen (Typ III) in Körperflüssigkeiten**
Monoclonal antibody for the selective immunological determination of intact procollagen peptide (type III) and procollagen (type III) in body fluids
Anticorps monoclonal pour la détermination immunologique sélective de procollagène peptide intact (type III) et procollagène (type III) dans les fluides corporels

(30) Priorität: 02.05.1987 DE 3714633
(43) Veröffentlichungstag der Anmeldung: 09.11.1988
(73) Patentinhaber: HOECHST AKTIENGESELLSCHAFT, 65926 Frankfurt am Main (DE); Max-Planck-Gesellschaft zur Förderung der Wissenschaften e.V., D-37073 Göttingen (DE)
(72) Erfinder: Brocks, Dietrich, Dr., D-6200 Wiesbaden (DE); Pünter, Jürgen, Dr., D-6238 Hofheim am Taunus (DE); Strecker, Helmut, Dr., Verstorben (DE); Timpl, Rupert, Dr., D-8035 Gauting (DE)

(56) Entgegenhaltungen:
- CLIN. CHEM., Band 31, Nr. 8, 1985, Seiten 1301-1304; O. NIEMELÄ: "Radioimmunoassays for type III procollagen amino-terminal peptides in humans"

## Beschreibung

Prokollagen Peptid (Typ III) ist das aminoterminale Propeptid des Kollagen (Typ III), das nach Sezernierung des Prokollagen (Typ III)-Moleküls extrazellulär abgespalten wird. Mit einer radioimmunologischen Bestimmungsmethode wie sie in der Europäischen Patentschrift Nr. 4940 beschrieben ist, kann die Konzentration dieses Prokollagen Peptids in Körperflüssigkeiten bestimmt werden. Die Kenntnis der Serumkonzentration des Peptids erlaubt Aussagen über die Aktivität fibrotischer Erkrankungen, wie z.B. der Leber [Rohde, H. et al. Eur. J. Clin. Invest. 9, 451 - 459 (1979)].

Die exakte, selektive Bestimmung von Prokollagen Peptid (Typ III) in Serum und anderen Körperflüssigkeiten ist mit den bisher beschriebenen Methoden aber nicht möglich, da die polyklonalen Antikörper, die in diesen Methoden eingesetzt werden, mit verschiedenen, im Serum vorkommenden Antigenen, die zum Teil Abbauprodukte des Prokollagen Peptids (Typ III) darstellen, mit unterschiedlicher, niedrigerer Affinität reagieren [Niemelä, O. et al. Clin. Chim. Acta 124, 39 - 44 (1982)]. Dies führt dazu, daß die Serumverdünnungskurven und die Verdünnungskurven anderer Körperflüssigkeiten zur Eichkurve, die mit reinem Prokollagen Peptid (Typ III) erstellt wird, nicht parallel sind und daher von jeder unbekannten Probe der Antigengehalt in mehreren Verdünnungen bestimmt werden muß, um die Antigenkonzentration über den 50 % Interzept der Verdünnungskurve zu ermitteln.

Mit Hilfe des Verfahrens der Europäischen Patentanmeldung 0089008 ist es möglich, dieses technische Problem zu lösen, indem Antikörper eingesetzt werden, die für intaktes Prokollagen Peptid (Typ III) und sein Abbauprodukt Col 1 vergleichbare Affinität haben. Mit diesem Verfahren werden intaktes und degradiertes Prokollagen Peptid (Typ III) gemeinsam bestimmt, was jedoch zur Ungenauigkeit in der diagnostischen Aussage führt, da Normalkollektiv und Patientenkollektiv stark überlappen können.

Überraschenderweise wurde nun ein monoklonaler Antikörper gefunden, der nicht mit den in Körperflüssigkeiten vorkommenden Abbauprodukten des Prokollagen Peptids (Typ III) reagiert. Mit diesem monoklonalen Antikörper ist eine immunologische Bestimmung des aminoterminalen Prokollagen Peptids (Typ III) ermöglicht, bei der dessen Abbauprodukte nicht miterfaßt werden, und die sich dadurch auszeichnet, daß Serumverdünnungskurven, die Verdünnungskurven anderer Körperflüssigkeiten und die Eichkurve komplette Parallelität zeigen.

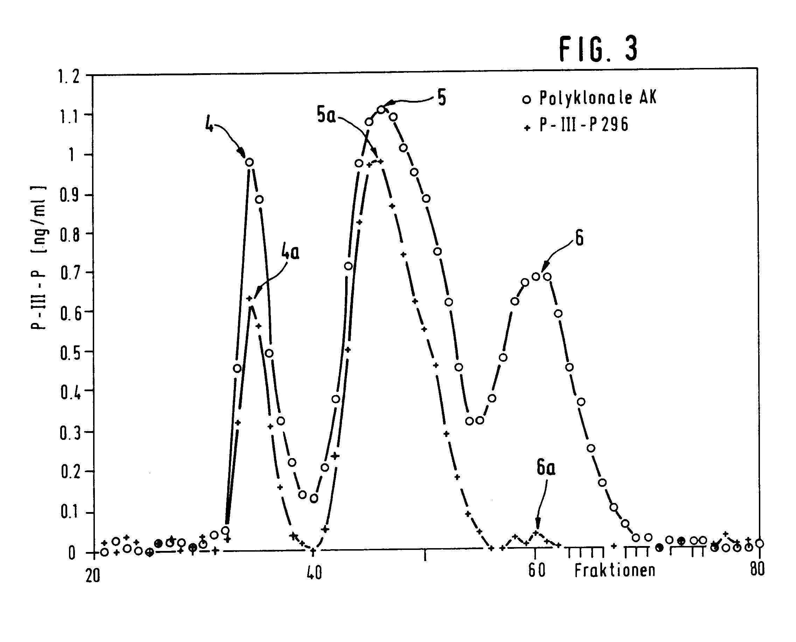

Die Erfindung betrifft somit:
1. Einen monoklonalen Antikörper mit dem in Figur 3 dargestellten Reaktionsmuster gegenüber intaktem Prokollagen (Typ III) und pN-Kollagen (Prokollagen, dem das C-terminale Propeptid fehlt, Peak 4a), intaktem aminoterminalen Prokollagen Peptid (Typ III) (Peak 5a) sowie Col 1 und Abbauprodukten des aminoterminalen Prokollagen Peptids (Typ III) mit gleichem Molekulargewicht wie Col 1 (Peak 6a).
2. Eine Hybridoma Zellinie, die durch Fusion von Zellen aus einer Myelom Linie und Lymphozyten von einem zuvor mit Prokollagen Peptid (Typ III) immunisierten Tier gebildet wird und die den unter 1. charakterisierten Antikörper produziert.
3. Ein Verfahren zur Herstellung des unter 1. charakterisierten Antikörpers.
4. Ein Verfahren zur quantitativen immunologischen Bestimmung von Prokollagen Peptid (Typ III) mit dem unter 1. charakterisierten Antikörper.
5. Eine diagnostische Zusammensetzung zur Feststellung der Prokollagen Peptid (Typ III)-Menge in Körperflüssigkeiten, die aus einer wirksamen Menge des unter 1. charakterisierten monoklonalen Antikörpers im Gemisch mit einem diagnostisch annehmbaren Träger besteht.

Im folgenden wird die Erfindung detailliert erläutert, insbesondere die bevorzugten Ausführungsformen. Ferner wird die Erfindung in den Patentansprüchen definiert.

Zur Herstellung der monoklonalen Antikörper können Tiere, bevorzugt Nagetiere, wie z.B. Mäuse, Ratten, Kaninchen, Meerschweinchen, mit Prokollagen Peptid (Typ III), das nach dem Verfahren des Europäischen Patents 4940 isoliert wurde, in Gegenwart von Adjuvans immunisiert werden. Besonders bevorzugt werden Mäuse eingesetzt, insbesondere solche vom SJL Stamm. Mit wiederholten Sekundärinjektionen, beispielsweise im Abstand von 4 bis 8 Wochen, wird die Immunantwort verstärkt. Der Erfolg der Immunisierung wird durch Bestimmung der Konzentration von Antikörpern im radioimmunologischen Bindungstest [R. Timpl und L. Risteli, Immunochemistry of the extracellular matrix, H. Furthmayr Ed., Vol. 1, 199 (1982)] kontrolliert. Einige Tage vor Fusion der Lymphozyten mit einer Myelom Zellinie werden die Tiere mit Prokollagen Peptid (Typ III) ohne Adjuvans behandelt. Lymphozyten der Tiere werden gewonnen und mit einer Myelom Zellinie fusioniert, die ebenfalls von einer der oben genannten Tierspezies stammen kann, vorzugsweise jedoch von der Maus, insbesondere mit der Zellinie P3X63AG8.653. Es werden vorteilhaft Lymphozyten mit Myelom-Zellinien gleicher Spezies fusioniert. Die Fusion und weitere Züchtung der Zellklone werden in einer dem Fachmann bekannten Weise durchgeführt, wobei im Überstand der Zellkultur mittels immunologischer Bindungstests die Konzentration spezifischer Antikörper bestimmt wird. Aus den aus der Fusion hervorgehenden Zellklonen werden geeignete Klone für die Verwendung in immunologischen Verfahren ausgewählt. Besonders bevorzugt wird mit einer Zellinie gearbeitet, die durch Fusion von Lymphozyten von Mäusen des SJL-Stamms gegen Prokollagen Peptid Typ III mit der Maus Myelom-Zellinie P3X63AG8.653 hergestellt wird. Diese Zellinie ist bei European Collection of Animal Cell Cultures (ECACC), PHLS Centre for Applied Microbiology and Research, Porton Down, Salisbury, SP4 0JG, U.K. unter der Nummer 87042308 hinterlegt.

Die erfindungsgemäßen monoklonalen Antikörper gehören in die Gruppe der Immunglobuline, bevorzugt in die Klasse der IgG-, IgA- und IgM-Proteine. Antikörper der IgG Klasse, insbesondere der Subklasse IgG2b sind besonders vorteilhaft einsetzbar. Der erfindungsgemäße Antikörper ist insbesondere deshalb überraschend, weil seine Affinität zum Antigen höher ist als die Affinität polyklonaler Antikörper. Üblicherweise wird das Gegenteil gefunden. Verdeutlicht werden die Eigenschaften der monoklonalen Antikörper anhand des aus der Zellinie ECACC 87042308 gewonnenen monoklonalen Antikörpers PIIIP 296, wenn die im Serum vorhandenen Antigene über Gelfiltrationschromatographie nach ihrem Molekulargewicht aufgetrennt werden und die Fraktionen der Chromatographie im Radioimmunoassay eingesetzt werden. Dabei zeigt sich, daß die Antigenfraktion, die bei Analyse mit polyklonalen Antikörpern miterfaßt wird und das Molekulargewicht des Abbauprodukts Col 1 hat, von dem erfindungsgemäßen monoklonalen Antikörper nicht erfaßt wird. In Figur 3 ist das Elutionsprofil der Gelfiltrationschromatographie von humanem Serum, wie es der monoklonale Antikörper zeigt, im Vergleich zum Elutionsprofil, wie es polyklonale Antikörper aufweisen, gezeigt. Peak 4/4a entspricht intaktem Prokollagen Typ III bzw. pN-Kollagen Typ III [Rohde H. et al. Eur. J. Biochem. 135, 197 [1983)]. Peak 5/5a entspricht intaktem aminoterminalem Prokollagen Peptid Typ III (P III P), während Peak 6/6a Col 1 entspricht sowie Abbauprodukten des aminoterminalen Prokollagen Peptids Typ III mit gleichem Molekulargewicht wie Col 1 [Rohde H. et al. Eur. J. Biochem 135, 197 (1983)]. Die Konzentration der Stoffe in den entsprechenden Fraktionen kann mit Hilfe einer Prokollagen Peptid Typ III Eichkurve bestimmt werden.

Für die Herstellung der erfindungsgemäßen Antikörper ist es wichtig, daß eine geeignete Quelle zur Gewinnung des Antigens zur Verfügung steht. Wie schon erwähnt, wird humanes oder tierisches, hochgereinigtes Prokollagen Peptid (Typ III) vorteilhaft nach dem Verfahren des Europäischen Patents 4940 isoliert, indem Gewebe oder pathologische Körperflüssigkeiten mit Kollagenase abgebaut werden und das Prokollagen Peptid aus der Reaktionslösung abgetrennt und durch Kombination von chromatographischen Methoden und/oder Immunadsorbtion gereinigt wird.

Der erfindungsgemäße monoklonale Antikörper kann in verschiedenen immunologischen Verfahren, einschließlich allen Formen des Radioimmunassays, z.B. sequentielle Sättigungsanalyse oder Gleichgewichtsanalyse, ggf. nach Markierung mit Chloramin T oder Bolton-Hunter-Reagenz [Felber, Meth. Biochem. Anal. 22, 1 (1974); Shelley et al., Clin. Chem. 19, 146 (1975)] sowie in anderen kompetitiven und nicht-kompetitiven Bindungsassays, wie Fluoreszens-, Enzym-, Chemiluminestenz- oder anderen Immunoassays, einschließlich Immunoradiometric Assay (IRMA) verwendet werden. Der monoklonale Antikörper kann daher in immunologischen Verfahren zur Isolierung und Charakterisierung sowie zur quantitativen Bestimmung von Prokollagen Peptid (Typ III) in Geweben und Körperflüssigkeiten eingesetzt werden. Man verfährt nach den dem Fachmann an sich bekannten Methoden, indem eine flüssige Probe, die Prokollagen Peptid (Typ III) enthält, mit dem erfindungsgemäßen monoklonalen Antikörper, sei es in Lösung oder auf einem festen Träger, zur Reaktion gebracht wird und die Menge des Prokollagen Peptids (Typ III) über den gebildeten Antigen-Antikörper-Komplex bestimmt wird. Dabei spielt es keine Rolle, ob das Prokollagen Peptid (Typ III) noch mit dem Aminoterminus des Prokollagen (Typ III) verknüpft ist oder nicht. Die bislang bei der immunologischen Bestimmung störenden Abbauprodukte des Prokollagen Peptids (Typ III), insbesondere das Col 1, werden von dem erfindungsgemäßen Antikörper nicht miterfaßt.

In den folgenden Beispielen wird die Erfindung weitergehend erläutert. Prozentangaben beziehen sich auf das Gewicht, wenn nicht anders angegeben.

### Beispiel 1

### Herstellung von Prokollagen-Peptid (Typ III) (P III P):

Prokollagen-Peptid (Typ III) wird hergestellt durch Einwirkung von Kollagenase auf Prokollagen (Typ III) bei 37°C. Hierbei wird das Peptid keinen Denaturierungsmitteln ausgesetzt. Zur Herstellung größerer Mengen des Peptids wird ein modifiziertes Verfahren angewendet. Alle Verfahrensschritte bis zur Einwirkung der Kollagenase werden im Kälteraum durchgeführt. Die verschiedenen NaCl-Lösungen, die zur Löslichmachung eingesetzt werden, enthalten 0,05 M Tris-HCl, pH 7,4, 0,01 M EDTA, Natriumazid (200 mg/ml) und die Protease-Inhibitoren Phenylmethylsulfonylfluorid (3 µg/ml) und p-Chlormercuribenzoat (3 µg/ml).

Foetale Kälberhaut (3 kg) wird in 10 l 1 M NaCl homogenisiert und zwei Tage extrahiert. Gelöstes Kollagen wird aus der Extraktionslösung gefällt durch Zugabe von festem NaCl bis zu einer Endkonzentration von 2,5 M. Nach Rühren über Nacht wird das Präzipitat durch Zentrifugation gesammelt (1800 x g, 20 Minuten), zweimal mit 2,5 M NaCl gewaschen und wieder aufgelöst, indem es über Nacht in 10 l 0,5 M NaCl gerührt wird. Kleine Mengen unlöslichen Materials werden durch Zentrifugation entfernt. Die so erhaltene Mischung von Kollagen (Typ III) und Prokollagen (Typ III) wird mit 1,6 M NaCl gefällt. Das Präzipitat wird dann in 2 l 0,05 M Tris-HCl (pH 8,0) suspendiert und nach Zusatz von 0,02 M CaCl₂ 20 Minuten bei 50°C erhitzt und anschließend 3 Stunden bei 37°C zusammen mit 1500 U bakterieller Kollagenase (CLSPA, Worthington, USA) pro Gramm feuchtes Präzipitat inkubiert. Nach Einwirkung der Kollagenase wird das gebildete Präzipitat durch Zentrifugation abgetrennt und die Lösung dialysiert gegen 0,005 M Tris-HCl, pH 8, 6,8 M Harnstoff und über eine DEAE-Cellulosesäule (5,0 x 30 cm) gegeben, die mit dem gleichen Puffer äquilibriert wurde.

Die auf der Säule gebundenen Proteine werden mit NaCl-Lösungen ausgewaschen, deren Konzentration von 0 bis 0,3 M ansteigt. Die gesamte Elutionsmenge beträgt 2 l. Die aus der Säule ausfließende Lösung wird bezüglich der Adsorption bei 236 nm und ihrer Antigenaktivität durch Verwendung von Antikörpern überprüft, die spezifisch für das aminoterminale Segment des Prokollagen (Typ III) sind. Normalerweise enthält der letzte Peak, der aus der Säule eluiert wird, das Prokollagen-Peptid (Typ III). Das Peptid wird durch Dialyse gegen destilliertes Wasser entsalzt und lyophilisiert. Die weitere Reinigung erfolgt auf einer Säule mit Agarose A 1,5 M (2 x 120 cm), die mit 1 M CaCl₂, 0,05 M Tris-HCL, pH 7,5 äquilibriert ist.

### Beispiel 2

### Hybridoma Produktion

Mäuse vom SJL-Stamm werden mit 5 µg Prokollagen Peptid (Typ III), das nach Beispiel 1 erhalten wurde, in Gegenwart von komplettem Freund'schen Adjuvans intramuskulär immunisiert. Nach 4 Wochen und nach drei Monaten wird die Immunreaktion durch eine weitere intramuskuläre Injektion von 5 µg Prokollagen Peptid (Typ III) in Gegenwart von inkompletten Freund'schen Adjuvans verstärkt. Drei Tage vor der Fusion wird die Immunantwort durch intraperitoneale Injektion von weiteren 50 µg Prokollagen Peptid (Typ III) verstärkt.

Zur Fusion werden die Tiere getötet und die Milzzellen isoliert. In Gegenwart von Polyethylenglykol werden die Milzzellen mit der Myeloma Zellinie P3X63AG8.653 fusioniert. Durch Kultivierung der Fusionsmischung in Hypoxanthin-Aminopterin-Thymidin-Medium über einen Zeitraum von zwei Wochen wird auf Milzzell x P3X63AG8.653-Hybride selektioniert. Zur Erreichung einer stabilen Zellinie werden die erhaltenen Zellklone mehrfach subkloniert. Die entstandenen Zellkolonien werden im radioimmunologischen Bindungstest auf Antikörperproduktion getestet. Die entstandene Zellinie, aus der man den monoklonalen Antikörper P III P 296 gewinnt, ist bei ECACC unter der Nummer 87042308 hinterlegt.

### Beispiel 3

### Radioimmun-Bindungstest

300 µl Zellkulturüberstand oder andere Probe, wie z.B. Aszites nach Züchtung von Zellen in der Bauchhöhle von Mäusen, werden mit 100 µl einer ¹²⁵J Prokollagen Peptid (Typ III) Lösung (1 ng Protein/100 µl hergestellt wie in EP 4940, Beispiel 1, beschrieben) über Nacht inkubiert. Die gebildeten Antigen-Antikörper Komplexe werden durch Zugabe von Anti Maus IgG Serum vom Schaf oder einer anderen Spezies ausgefällt. Nach Zentrifugation und Dekantieren des Überstands wird die Menge der präzipitierten Radioaktivität im γ-Szintillationsspektrometer bestimmt.

### Beispiel 4

### Radioimmun Test

0,2 ml der zu analysierenden Probe oder des Prokollagen Peptid (Typ III)-Standards werden mit einer in Bezug auf die Menge des markierten Antigens limitierenden Menge von PIIIP 296 (in 0,1 ml Puffer) und 0,1 ml ¹²⁵J-markiertem Prokollagen Peptids (Typ III) (enthält 1 ng Protein) über Nacht bei 4°C inkubiert. Anschließend wird mit einer vorher ausgetesteten Menge Anti Maus IgG Serum vom Schaf in Gegenwart von 10 % Polyethylenglykol (PEG 6000) 1 h inkubiert. Die gefällten Antigen-Antikörper-Komplexe werden abzentrifugiert (1500 x g) und nach Dekantieren wird im γ-Szintillationsspektrometer die Radioaktivität bestimmt.

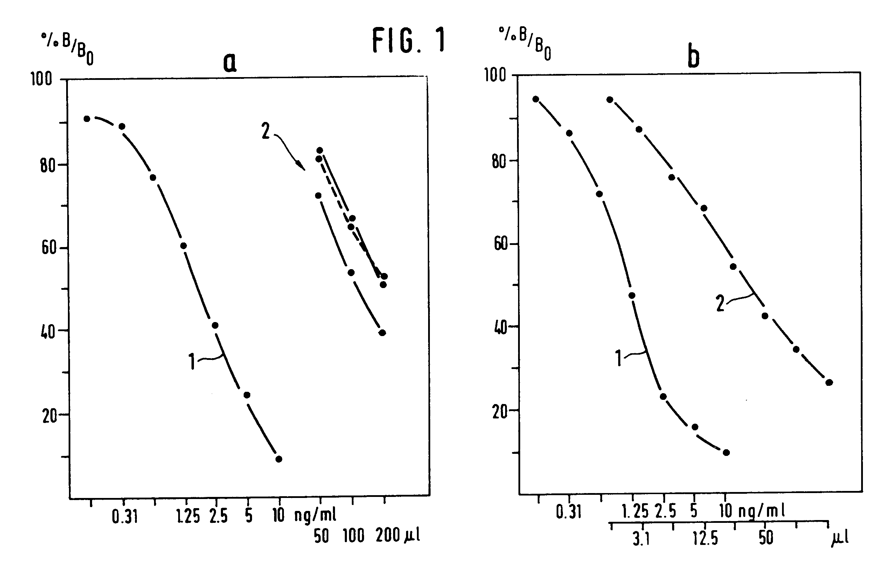

Durch Vergleich mit einer Eichkurve, zu deren Erstellung Standards mit unterschiedlichen Mengen Prokollagen Peptid (Typ III) eingesetzt werden, kann dann die Konzentration von Prokollagen Peptid (Typ III) in der unbekannten Lösung bestimmt werden. Figur 1 zeigt die Eichkurve (1) und Serumverdünnungskurven (2) mit PIIIP 296 (a) im Vergleich zum Verfahren nach EP 4940 mit polyklonalen Antikörpern (b).

### Beispiel 5

### Radioaktive Markierung des P III P 296

0,2 ml einer Lösung mit 0,2 mg des monoklonalen P III P 296 in 0,05 M Phosphatpuffer pH 7,4 werden in einem Polystyrol-Teströhrchen (12 x 55 mm) vorgelegt und mit 100 MBq Na¹²⁵J-Lösung, abgepuffert mit 10 µl 0,5 M Phosphatpuffer pH 7,4, versetzt. Nach Zusatz von 50 µl einer wäßrigen Lösung von 20 µg Chloramin T wird 1 min gemischt. Anschließend wird die Jodierungsreaktion durch Zugabe von 50 µl einer wäßrigen Lösung von 20 µg Natriumdisulfit beendet.

Das nicht umgesetzte Na¹²⁵J wird anschließend von dem ¹²⁵J-markierten P III P 296 durch Chromatographie an einem Anionenaustauscher abgetrennt. Die chromatographischen Fraktionen, die den aufgereinigten ¹²⁵J-markierten Antikörper enthalten, werden mit einer Lösung aus 20 g Tween 20 und 14,6 g Na₂EDTA in einem Liter 0,05 M Tris-HCl-Puffer pH 8,0 verdünnt, so daß die Konzentration des markierten Antikörpers 200 µg/l beträgt.

### Beispiel 6

### Antikörperbeschichtung von Teströhrchen

Zur Fixierung der Antikörper auf Polystyrolteströhrchen (12 x 75 mm) werden in jedes Röhrchen 300 µl einer Lösung von 4 mg monoklonalem P III P 296 pro Liter 0,01 M Natriumphosphatpuffer pH 6,4 gegeben und über Nacht bei Raumtemperatur inkubiert. Anschließend wird die Antikörperlösung abgesaugt. Danach werden in jedes Röhrchen 500 µl einer 1%igen Lösung von Rinderserumalbumin in 0,05 M Tris-Citrat-Puffer pH 7,5 gegeben und über Nacht bei Raumtemperatur inkubiert. Anschließend wird die Lösung abgesaugt. Die Antikörper-beschichteten Röhrchen werden über Silikagel getrocknet.

### Beispiel 7

### Immunradiometrischer Test

0,1 ml der zu analysierenden Probe oder 0,1 ml des Prokollagen (Typ III)-Standards werden in Polystyrol-Teströhrchen, die zuvor mit 1,2 µg des monoklonalen P III P 296 beschichtet wurden, unter Zusatz von 0,1 ml phosphatgepufferte Kochsalzlösung (phosphate buffered saline, PBS) bei Raumtemperatur 2 Stunden inkubiert. Anschließend werden die Teströhrchen zweimal mit je 1 ml PBS gewaschen und dekantiert. Danach werden in die Röhrchen 200 µl 125-J-markierter monoklonaler P III P-Antikörper (enthält 40 ng Antikörper) gegeben und 2 Stunden bei Raumtemperatur inkubiert. Die Radioaktivität der an der Teströhrchenwand gebundenen Antikörper-Antigen-¹²⁵J-Antikörperkomplexe wird nach zweimaligem Waschen mit je 1 ml PBS und anschließendem Dekantieren im Szintillationsmeßgerät bestimmt.

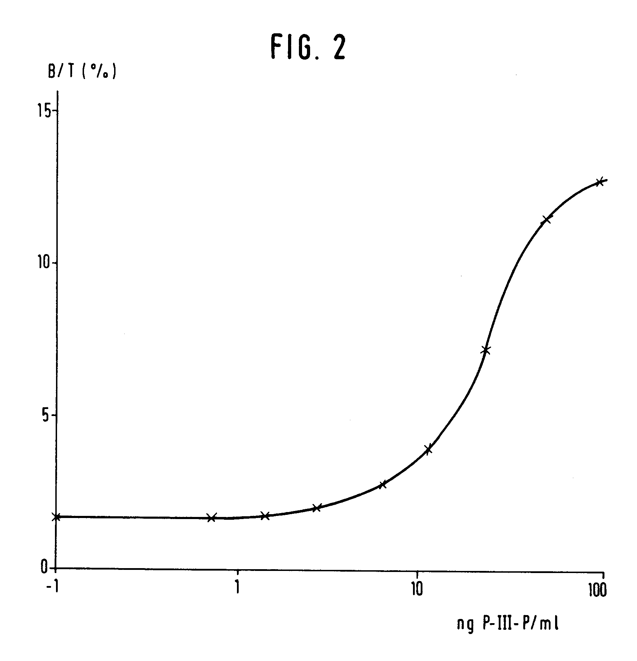

Durch Vergleich mit einer Eichkurve, zu deren Einstellung Standards mit unterschiedlichen Mengen Prokollagen-Peptid (Typ III) eingesetzt werden, kann dann die Konzentration von Prokollagen-Peptid (Typ III) in der unbekannten Probenlösung berechnet werden. Die Fig. 2 zeigt die Eichkurve des radioimmunometrischen Assays. (B/T bedeutet: Antikörper gebunden/gesamt).

### Beispiel 9

### Bestimmung der Molekulargewichtsverteilung der mit PIIIP 296 reagierenden Antigene in humanem Serum

1 ml Serum werden per Gelfiltrationschromatographie über ein Allyldextran vernetzt mit N,N'-Methylenbisacrylamid [®Sephacryl S 300 Säule (1,6 x 130 cm)], äquilibriert in "Phosphate buffered Saline" (PBS) mit 0,04 % eines nichtionischen Detergens, beispielsweise eines polyethoxylierten Sorbitan-Monolaurats (Tween 20), in Fraktionen mit 3,3 ml aufgetrennt. Je 0,2 ml der Fraktionen werden in den Radioimmuntest nach Beispiel 4 eingesetzt. Figur 3 zeigt das Elutionsprofil des mittels PIIIP 296 bestimmten Antigens im Vergleich zum Profil des mit Hilfe der polyklonalen Antikörper bestimmten Antigens:
- Peak 4/4a: entspricht pN Kollagen und intaktem aminoterminalem Prokollagen (Typ III)
- Peak 5/5a: entspricht aminoterminalem Prokollagen Peptid (Typ III)=(P III P)
- Peak 6/6a: entspricht Col 1 sowie Abbauprodukten des aminoterminalen Prokollagen Peptids (Typ III) mit gleichem Molekulargewicht wie Col 1.

Die Konzentration der Stoffe in den entsprechenden Fraktionen kann mit Hilfe einer Prokollagen Peptid (Typ III) Eichkurve bestimmt werden.

## Patentansprüche (Patentansprüche für folgende(n) Vertragsstaat(en): AT, BE, CH, DE, FR, GB, IT, LI, LU, NL, SE)

1. Monoklonaler Antikörper mit dem in Figur 3 dargestellten Reaktionsmuster gegenüber intaktem Prokollagen (Typ III) und pN-Kollagen (Peak 4a), intaktem aminoterminalen Prokollagen Peptid (Typ III) (Peak 5a) sowie Col 1 und Abbauprodukten des aminoterminalen Prokollagen Peptids (Typ III) mit gleichem Molekulargewicht wie Col 1 (Peak 6a).

2. Monoklonaler Antikörper nach Anspruch 1 mit spezifischer Wirkung gegen ein Epitop des aminoterminalen Prokollagen Peptids (Typ III), das in Col 1 nicht vorhanden ist.

3. Monoklonaler Antikörper nach Anspruch 1 oder 2, dadurch gekennzeichnet, daß er der Klasse IgG zugeordnet wird.

4. Monoklonaler Antikörper nach einem oder mehreren der Ansprüche 1 bis 3, dadurch gekennzeichnet, daß er durch ein Hybridom gebildet wird, das durch Fusion von Zellen einer Myelom Linie und von Lymphozyten eines zuvor mit Prokollagen Peptid Typ III immunisierten Tieres entstanden ist.

5. Monoklonaler Antikörper PIIIP 296 der Hybridoma Zellinie ECACC 87042308.

6. Hybridoma Zellinie, die einen Antikörper gemäß der Ansprüche 1 bis 5 produziert, gebildet durch Fusion von Zellen aus einer Myelom-Zellinie und von Lymphozyten eines zuvor mit Prokollagen Peptid (Typ III) immunisierten Tieres.

7. Die Hybridoma Zellinie ECACC 87042308.

8. Verfahren zur Herstellung eines monoklonalen Antikörpers gegen aminoterminales Prokollagen Peptid (Typ III), dadurch gekennzeichnet, daß
a) Tiere mit aminoterminalem Prokollagen Peptid (Typ III) immunisiert werden,
b) Lymphozyten gewonnen und mit Myelom-Zellen fusioniert werden,
c) die Hybride hinsichtlich des Vorliegens eines Antikörpers mit den in einem der Ansprüche 1 bis 5 angegebenen Eigenschaften ausgewählt und geklont werden und
d) der Antikörper aus den genannten Klonen gewonnen wird.

9. Verfahren nach Anspruch 8, dadurch gekennzeichnet, daß zur Ausführung des Schritts d) die Hybridoma Zellinie ECACC 87042308 eingesetzt wird.

10. Verfahren zur quantitativen immunologischen Bestimmung von Prokollagen Peptid (Typ III) mit Antikörpern, dadurch gekennzeichnet, daß
a) eine flüssige Probe, die aminoterminales Prokollagen Peptid (Typ III) oder Prokollagen (Typ III) enthält, mit einem monoklonalen Antikörper nach einem oder mehreren der Ansprüche 1 bis 5 zur Reaktion gebracht wird und
b) die Menge des aminoterminalen Prokollagen Peptids (Typ III) bzw. das Prokollagen über den gebildeten Antigen-Antikörper-Komplex bestimmt wird.

11. Diagnostische Zusammensetzung zur Feststellung der Prokollagen Peptid (Typ III)-Menge in Körperflüssigkeiten, gekennzeichnet durch eine wirksame Menge des monoklonalen Antikörpers nach einem oder mehreren der Ansprüche 1 bis 5 im Gemisch mit einem diagnostisch annehmbaren Träger.

## Patentansprüche (Patentansprüche für folgende(n) Vertragsstaat(en): ES, GR)

1. Verfahren zur Herstellung eines monoklonalen Antikörpers gegen aminoterminales Prokollagen Peptid (Typ III), dadurch gekennzeichnet, daß
a) Tiere mit aminoterminalem Prokollagen Peptid (Typ III) immunisiert werden,
b) Lymphozyten gewonnen und mit Myelom-Zellen fusioniert werden,
c) die Hybride hinsichtlich des Vorliegens eines Antikörpers mit den in Abb. 3 angegebenen Eigenschaften ausgewählt und geklont werden, wobei der monoklonale Antikörper spezifische Wirkung gegen ein Epitop des aminoterminalen Prokollagen Peptids (Typ III) besitzt, welches nicht in Col 1 vorhanden ist, der monoklonale Antikörper der Klasse IgG zugeordnet wird und durch ein Hybridom gebildet wird, daß durch Fusion von Zellen einer Myelom Linie und von Lymphozyten eines zuvor mit Prokollagen Peptid Typ III immunisierten Tieres entstanden ist und der monoklonale Antikörper P III P296 der Hybridoma Zellinie ECACC 87042308 verwendet wird und
d) der monoklonale Antikörper mit dem in Figur 3 dargestellten Reaktionsmuster gegenüber intaktem Prokollagen (Typ III) und PN-Kollagen (Peak 4a), intaktem aminoterminalen Prokollagen Peptid (Typ III) (Peak 5a) sowie Col 1 und Abbauprodukten des aminoterminalen Prokollagen Peptids (Typ III) mit gleichem Molekulargewicht wie Col 1 (Peak 6a) aus den genannten Klonen gewonnen wird.

2. Verfahren nach Anspruch 1, dadurch gekennzeichnet, daß zur Ausführung des Schritts d) die Hybridoma Zellinie ECACC 87042308 eingesetzt wird.

3. Verfahren zur quantitativen immunologischen Bestimmung von Prokollagen Peptid (Typ III) mit Antikörpern, dadurch gekennzeichnet, daß
a) eine flüssige Probe, die aminoterminales Prokollagen Peptid (Typ III) oder Prokollagen (Typ III) enthält, mit dem nach Anspruch 1 erhältlichen monoklonalen Antikörper zur Reaktion gebracht wird und
b) die Menge des aminoterminalen Prokollagen Peptids (Typ III) bzw. das Prokollagen über den gebildeten Antigen-Antikörper-Komplex bestimmt wird.

## Claims (Claims for the following Contracting State(s): AT, BE, CH, DE, FR, GB, IT, LI, LU, NL, SE)

1. A monoclonal antibody having the reaction pattern which is depicted in Figure 3 towards intact procollagen (type III) and pN collagen(peak 4a), intact amino-terminal procollagen peptide (type III) (peak 5a), and Col 1 and breakdown products of the amino-terminal procollagen peptide (type III) having the same molecular weight as Col 1 (peak 6a).

2. A monoclonal antibody as claimed in claim 1, having a specific action against an epitope of amino-terminal procollagen peptide (type III) which is not present in Col 1.

3. A monoclonal antibody as claimed in claim 1 or 2, which is assigned to the class IgG.

4. A monoclonal antibody as claimed in one or more of claims 1 to 3, which is formed by a hydridoma which is produced by fusion of cells of a myeloma line and of lymphocytes from an animal which has previously been immunized with procollagen peptide type III.

5. A monoclonal antibody PIIIP 296 of the hybridoma cell line ECACC 87042308.

6. A hybridoma cell line which produces an antibody as claimed in claims 1 to 5, formed by fusion of cells of a myeloma cell line and of lymphocytes from an animal which has previously been immunized with procollagen peptide (type III).

7. The hybridoma cell line ECACC 87042308.

8. A process for the preparation of a monoclonal antibody against amino-terminal procollagen peptide (type III), which comprises
a) immunization of animals with amino-terminal procollagen peptide (type III),
b) isolation of lymphocytes and fusion thereof with myeloma cells,
c) selection of the hybrids for the presence of an antibody having the properties indicated in one of claims 1 to 5, and cloning thereof, and
d) isolation of the antibodies from the said clones.

9. The process as claimed in claim 8, wherein the hybridoma cell line ECACC 87042308 is used for carrying out step d).

10. A method for the quantitative immunological determination of procollagen peptide (type III) using antibodies, which comprises
a) reacting a liquid sample which contains amino-terminal procollagen peptide (type III) or procollagen (type III) with a monoclonal antibody as claimed in one or more of claims 1 to 5, and
b) determining the amount of amino-terminal procollagen peptide (type III) or the procollagen via the antigen/antibody complex which is formed.

11. A diagnostic composition for establishing the amount of procollagen peptide (type III) in body fluids, which contains an effective amount of the monoclonal antibody as claimed in one or more of claims 1 to 5, mixed with a diagnostically acceptable vehicle.

## Claims (Claims for the following Contracting State(s): ES, GR)

1. A process for the preparation of a monoclonal antibody against amino-terminal procollagen peptide type (type III), which comprises
a) immunization of animals with amino-terminal procollagen peptide (type III),
b) isolation of lymphocytes and fusion thereof with myeloma cells,
c) selection of the hybrids for the presence of an antibody having the properties indicated in Figure 3, and cloning thereof, whereas the monoclonal antibody has a specific action against an epitope of aminoterminal procollagen peptide (type III) which is not present in Col 1, the monoclonal antibody is assigned to the class IgG and is formed by a hybridoma which is produced by fusion of cells of a myeloma line and of lymphocytes from an animal which has previously been immunized with procollagen peptide type III and the monoclonal antibody P III P 296 of the hybridoma cell line ECACC 87042308 is used, and
d) isolation of the monoclonal antibody having the reaction pattern which is depicted in Figure 3 towards intact procollagen (type III) and pN collagen (peak 4a), intact amino-terminal procollagen peptide (type III) (peak 5a), and Col 1 and breakdown products of the amino-terminal procollagen peptide (type III) having the same molecular weight as Col 1 (peak 6a) from the said clones.

2. The process as claimed in claim in 1, wherein the hybridoma cell line ECACC 87042308 is used for carrying out step d).

3. A method for the quantitative immunological determination of procollagen peptide (type III) using antibodies, which comprises
a) reacting a liquid sample which contains amino-terminal procollagen peptide (type III) or procollagen (type III) with the monoclonal antibody obtainable as claimed in claim 1, and
b) determining the amount of amino-terminal procollagen peptide (type III) or the procollagen via the antigen/antibody complex which is formed.

## Revendications (Revendications pour l'(les) Etat(s) contractant(s) suivant(s): AT, BE, CH, DE, FR, GB, IT, LI, LU, NL, SE)

1. Anticorps monoclonal ayant le profil réactionnel représenté dans la figure 3, dirigé contre le procollagène intact (type III) et le pN-collagène (pic 4a), le peptide aminoterminal intact du procollagène (type III) (pic 5a), et contre le Col 1 et les produits de décomposition du peptide aminoterminal du procollagène (type III) ayant la même masse moléculaire que le Col 1 (pic 6a).

2. Anticorps monoclonal selon la revendication 1, exerçant une action spécifique contre un épitope du peptide aminoterminal du procollagène (type III) qui n'est pas présent dans le Col 1.

3. Anticorps monoclonal selon la revendication 1 ou 2, caractérisé en ce qu'on l'affecte à la catégorie des IgG.

4. Anticorps monoclonal selon une ou plusieurs des revendications 1 à 3, caractérisé en ce qu'il est produit par un hybridome, lequel est formé par fusion de cellules provenant d'une lignée de myélome et de lymphocytes provenant d'un animal préalablement immunisé à l'aide de peptide de procollagène de type III.

5. Anticorps monoclonal PIIIP 296 de la lignée cellulaire d'hybridome ECACC 87042308.

6. Lignée cellulaire d'hybridome produisant un anticorps monoclonal selon les revendication 1 à 5, formée par fusion de cellules provenant d'une lignée cellulaire de myélome et de lymphocytes provenant d'un animal préalablement immunisé à l'aide de peptide de procollagène de type III.

7. Lignée cellulaire d'hybridome ECACC 87042308.

8. Procédé pour préparer un anticorps monoclonal dirigé contre le peptide aminoterminal du procollagène (type III), caractérisé en ce que :
a) on immunise des animaux à l'aide du peptide aminoterminal du procollagène (type III),
b) on récupère des lymphocytes et on les fait fusionner avec des cellules de myélome,
c) on sélectionne les hybrides d'après la présence d'un anticorps doté des propriétés citées dans l'une des revendications 1 à 5, et on les clone, et
d) on récupère l'anticorps dans les clones mentionnés.

9. Procédé selon la revendication 8, caractérisé en ce qu'on utilise la lignée cellulaire d'hybridome ECACC 87042308 pour effectuer l'étape d).

10. Procédé de détermination immunologique quantitative de peptide du procollagène (type III) avec des anticorps, caractérisé en ce que :
a) on met à réagir un échantillon liquide, contenant le peptide aminoterminal du procollagène (type III), ou du procollagène (type III), avec un anticorps monoclonal selon une ou plusieurs des revendications 1 à 5, et
b) on détermine la quantité de peptide aminoterminal du procollagène (type III), ou de procollagène, d'après le complexe antigène-anticorps formé.

11. Composition pour le diagnostic, destinée à établir la quantité de peptide du procollagène (type III) présente dans des liquides corporels, caractérisée par une quantité efficace de l'anticorps monoclonal selon une ou plusieurs des revendications 1 à 5, mélangée avec un véhicule absorbable du point de vue du diagnostic.

## Revendications (Revendications pour l'(les) Etat(s) contractant(s) suivant(s): ES, GR)

1. Procédé pour préparer un anticorps monoclonal dirigé contre le peptide aminoterminal du procollagène (type III), caractérisé en ce que:
a) on immunise des animaux à l'aide du peptide aminoterminal du procollagène (type III) ;
b) on récupère des lymphocytes et on les fait fusionner avec des cellules de myélome ;
c) on sélectionne les hybrides d'après la présence d'un anticorps choisi parmi un anticorps monoclonal ayant le profil réactionnel représenté dans la figure 3, dirigé contre le procollagène intact (type III) et le pN-collagène (pic 4a), le peptide aminoterminal intact du procollagène (type III) (pic 5a), et contre le Col 1 et les produits de décomposition du peptide aminoterminal du procollagène (type III) ayant la même masse moléculaire que le Col 1 (pic 6a) et éventuellement exerçant une action spécifique contre un épitope du peptide aminoterminal du procollagène (type III) qui n'est pas présent dans le Col 1 et éventuellement étant affecté à la catégorie des IgG et éventuellement étant produit par un hybridome, lequel est formé par fusion de cellules provenant d'une lignée de myélome et de lymphocytes provenant d'un animal préalablement immunisé à l'aide de peptide de procollagène de type III, et l'anticorps monoclonal PIIIP 296 de la lignée cellulaire d'hybridome ECACC 87042308 et on clone lesdits hybrides ; et
d) dans les clones mentionnés, on récupère l'anticorps monoclonal ayant le profil réactionnel représenté dans la figure 3, dirigé contre le procollagène intact (type III) et le pN-collagène (pic 4a), le peptide aminoterminal intact du procollagène (type III) (pic 5a), et contre le Col 1 et les produits de décomposition du peptide aminoterminal du procollagène (type III) ayant la même masse moléculaire que le Col 1 (pic 6a).

2. Procédé selon la revendication 1, caractérisé en ce qu'on utilise la ligne cellulaire d'hybridome ECACC 87042308 pour effectuer l'étape d).

3. Procédé de détermination immunologique quantitative de peptide du procollagène (type III) avec des anticorps, caractérisé en ce que :
a) on met à réagir un échantillon liquide, contenant le peptide aminoterminal du procollagène (type III), ou du procollagène (type III), avec l'anticorps monoclonal qu'on peut obtenir selon la revendication 1 ; et
b) on détermine la quantité de peptide aminoterminal du procollagène (type III), ou de procollagène, d'après le complexe antigène-anticorps formé.
